# FASCICULE DE BREVET EUROPEEN

(11) **EP 3 237 015 B1**
(45) Date de publication et mention de la délivrance du brevet: **01.05.2019**
(21) Numéro de dépôt: 15801727.7
(22) Date de dépôt: 20.11.2015
(51) Int. Cl.: A61K 47/50, C07F 9/38, C07C 335/04, A61P 19/00, A61P 35/00

(54) **DERIVES HYDROXYBISPHOSPHONIQUES HYDROSOLUBLES DE LA DOXORUBICINE**
WASSERLÖSLICHE BISPHOSPHONISCHE DOXORUBICINDERIVATE
HYDROSOLUBLE HYDROXYBISPHOSPHONIC DOXORUBICINE DERIVATIVES

(30) Priorité: 20.11.2014 FR 1461253
(43) Date de publication de la demande: 01.11.2017
(73) Titulaire: Atlanthera, 44800 Saint Herblain (FR)
(72) Inventeur: EGOROV, Maxim, 44340 Bouguenais (FR); GOUJON, Jean-Yves, 44590 Derval (FR); LE BOT, Ronan, 44100 Nantes (FR); DAVID, Emmanuelle, 44710 Port Saint Père (FR)
(74) Mandataire: Regimbeau
(86) Numéro de dépôt international: PCT/EP2015/077279
(87) Numéro de publication internationale: WO 2016/079327

(56) Documents cités:
- WO-A1-96/40156
- WO-A1-2009/083613
- WO-A1-2009/083614
- WO-A1-2012/130911
- WO-A2-2011/023367
- HOCHDÖRFFER KATRIN ET AL: "Development of novel bisphosphonate prodrugs of doxorubicin for targeting bone metastases that are cleaved pH dependently or by cathepsin B: synthesis, cleavage properties, and binding properties to hydroxyapatite as well as bone matrix.", JOURNAL OF MEDICINAL CHEMISTRY 13 SEP 2012, vol. 55, no. 17, 13 septembre 2012 (2012-09-13), pages 7502-7515, XP002736394, ISSN: 1520-4804
- SCOZZAFAVA A ET AL: "Carbonic anhydrase inhibitors. A general approach for the preparation of water-soluble sulfonamides incorporating polyamino-polycarboxylate tails and of their metal complexes possessing long-lasting, topical intraocular pressure-lowering properties", JOURNAL OF MEDICINAL CHEMISTRY, AMERICAN CHEMICAL SOCIETY, US, vol. 45, no. 7, 28 mars 2002 (2002-03-28), pages 1466-1476, XP002595850, ISSN: 0022-2623, DOI: 10.1021/JM0108202 [extrait le 2002-02-22]
- KHOLOD ET AL.: "Application of Quantum Chemical Approximations to Environmental Problems: Prediction of Water Solubility for Nitro Compounds", ENVIRONMENTAL SCIENCE & TECHNOLOGY, vol. 43, no. 24, décembre 2009 (2009-12), pages 9208-9215, XP002756683, ISSN: 0013-936X, DOI: 10.1021/ES902566B

## Description

La présente invention concerne des dérivés hydroxybisphosphoniques hydrosolubles de la doxorubicine ciblant le tissu osseux, notamment pour leur utilisation comme médicament, en particulier dans le traitement d'une tumeur osseuse, les procédés de synthèse de ceux-ci, et les compositions pharmaceutiques comprenant de tels dérivés.

Le tissu osseux est un tissu conjonctif en perpétuel remaniement composé de cristaux d'hydroxyapatite, d'une matrice extracellulaire et de cellules spécialisées (ostéoblastes et ostéoclastes). Tout dérèglement de l'équilibre entre les phénomènes d'apposition et résorption osseuse induit des pathologies ostéolytiques ou ostéocondensantes pouvant être d'origine tumorale (avec des tumeurs primaires, comme l'ostéosarcome, ou secondaires, comme les métastases osseuses). Les ostéosarcomes sont des tumeurs osseuses très agressives avec appositions périostées, destruction de la corticale et envahissement des parties molles. L'ostéosarcome survient généralement dans une population jeune (avec une médiane d'âge de 18 ans) et représente 5% des cancers de l'enfant. Ces tumeurs ont la particularité d'établir un cercle vicieux entre ostéolyse et prolifération tumorale. Le traitement actuel de ces tumeurs consiste en une chimiothérapie pré-opératoire, suivie par une exérèse chirurgicale de la tumeur, puis une chimiothérapie post-opératoire, permettant une survie des patients à 5 ans de 60-70% mais de seulement 30% lorsque des métastases pulmonaires sont détectées. Face à ces taux de survie assez médiocres, le développement de nouvelles thérapies pour les ostéosarcomes apparaît nécessaire.

Les dérivés d'acide hydroxybisphosphonique (HBP) sont des molécules connues pour leur très forte affinité pour le tissu osseux et leur propriété d'inhibition de la résorption osseuse (Moriceau et al., Curr Pharm Des. 2010, 16(27), 2981). La doxorubicine est un des produits essentiels utilisés dans la thérapie des tumeurs osseuses. Cependant la toxicité de la doxorubicine limite beaucoup son utilisation. L'intérêt d'associer chimiquement la doxorubicine à un HBP est double. La vectorisation doit permettre de cibler l'activité antitumorale de la doxorubicine au niveau du site osseux (et ainsi de traiter plus efficacement la tumeur) en diminuant la concentration circulante (permettant de diminuer sa toxicité). Le vecteur HBP peut en même temps améliorer le traitement d'ostéolyse tumorale par son effet anti-resorptif.

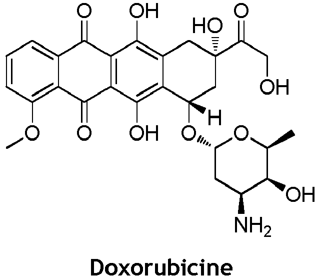

La demande WO 2012/130911 décrit notamment cette approche d'association chimique d'un vecteur avec des principes actifs, dont la doxorubicine, sur un vecteur portant une fonction HBP via une liaison imine. Toutefois, le produit décrit dans la demande WO 2012/130911 comportant un motif doxorubicine (modifié ou non) couplé à un vecteur HBP s'est révélé insoluble (ou très peu soluble sous sa forme modifiée) dans l'eau, même avec des additifs organiques (par ex. Tween®, PEG, glycérine, glucose) ou minéraux (par ex. carbonate ou bicarbonate de sodium), et dans les solvants organiques (par ex. l'éthanol ou le diméthylsuloxyde) ce qui rend difficile l'utilisation de ce produit comme médicament.

Il existe donc un besoin de développer de nouveaux dérivés de doxorubicine couplés à un vecteur HBP qui seraient solubles dans l'eau afin de permettre leur utilisation comme médicament.

La présente invention concerne donc un dérivé hydroxybisphosphonique de la doxorubicine de formule générale (I) suivante : 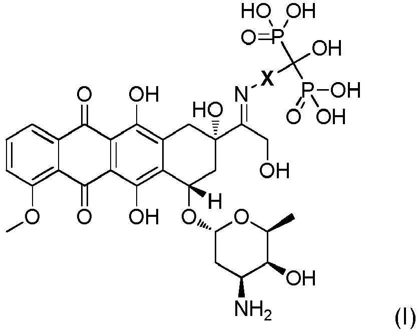 ou un sel pharmaceutiquement acceptable de celui-ci, dans laquelle X représente un groupe bivalent choisi parmi :
(1) 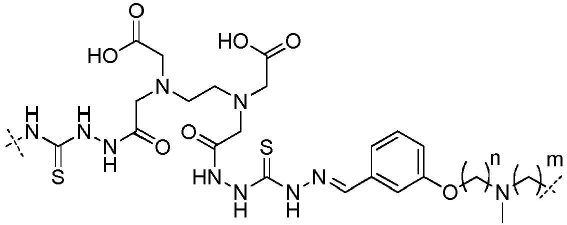 avec n et m représentant chacun, indépendamment l'un de l'autre, un nombre entier compris entre 1 et 6, notamment entre 1 et 4,
(2) 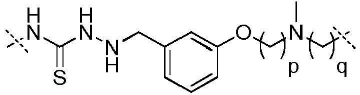 avec p et q représentant chacun, indépendamment l'un de l'autre, un nombre entier compris entre 1 et 6, notamment entre 1 et 4, et
(3) 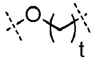 avec t représentant un nombre entier compris entre 1 et 6, notamment entre 1 et 4,
ces groupes étant liés à la fonction imine du composé de formule (I) via leur atome d'azote ou d'oxygène terminal et à la fonction acide hydroxybisphosphonique du composé de formule (I) via leur atome de carbone terminal.

Les composés de formule (I) selon la présente invention permettent donc une vectorisation de la doxorubicine (agent antitumoral) par un vecteur hydroxybisphosphonique ciblant le tissu osseux. Le type d'accroche de la doxorubicine et la nature du vecteur sont spécialement adaptés pour assurer la solubilité élevée des composés dans l'eau afin de faciliter leur utilisation clinique.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sel pharmaceutiquement acceptable » d'un composé, un sel qui est pharmaceutiquement acceptable, comme défini ici, et qui possède l'activité pharmacologique souhaitée du composé parent.

Les sels pharmaceutiquement acceptables comprennent notamment :
(1) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, et
(2) les sels d'addition de base pharmaceutiquement acceptable formés lorsqu'un proton acide présent dans le composé parent est soit remplacé par un ion métallique, par exemple un ion de métal alcalin (par ex. Na, K, Li ou Cs), un ion de métal alcalino-terreux (par ex. Ca, Mg) ou un ion d'aluminium ; soit coordonné avec une base organique pharmaceutiquement acceptable telle que la diéthanolamine, l'éthanolamine, N-méthylglucamine, la triéthanolamine, la trométhamine et similaires ; ou avec une base inorganique pharmaceutiquement acceptable telle que l'hydroxyde d'aluminium, l'hydroxyde de calcium, l'hydroxyde de potassium, le carbonate de sodium, le bicarbonate de sodium, l'hydroxyde de sodium et similaires.

Ainsi, n, m, p, q et t représentent chacun, indépendamment les uns des autres, un nombre entier égal à 1, 2, 3, 4, 5 ou 6, notamment égal à 1, 2, 3 ou 4.

Selon un mode de réalisation particulier de l'invention, n = 3, m = 2, p = 3, q = 2 et t = 1. Ainsi, avantageusement, X représente un groupe choisi parmi : 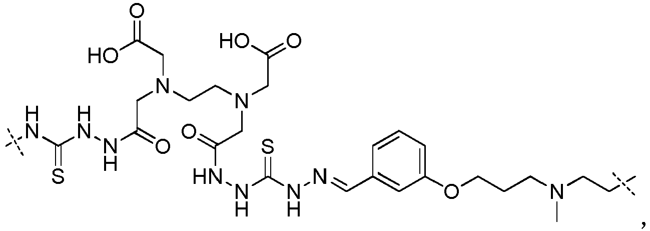 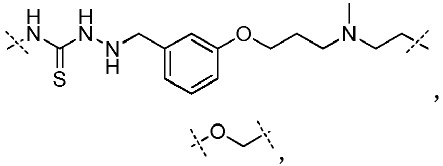 et ces groupes étant liés à la fonction imine du composé de formule (I) via leur atome d'azote ou d'oxygène terminal et à la fonction acide hydroxybisphosphonique du composé de formule (I) via leur atome de carbone terminal.

Selon un mode de réalisation particulier de l'invention, le composé de formule (I) est choisi parmi les composés (II), (III), (IV) décrits dans la partie expérimentale ci-après et leurs sels pharmaceutiquement acceptables.

La présente invention concerne également un composé de formule (I) tel que décrit ci-dessus ou un sel pharmaceutiquement acceptable de celui-ci, pour son utilisation en tant que médicament, notamment pour cibler le tissu osseux.

La présente invention concerne également l'utilisation d'un composé de formule (I) tel que décrit ci-dessus ou d'un sel pharmaceutiquement acceptable de celui-ci, pour la fabrication d'une composition pharmaceutique, ciblant plus particulièrement le tissu osseux.

En particulier, les composés de formule (I) selon l'invention pourront être utilisés pour le traitement d'une tumeur osseuse.

La présente invention concerne également une méthode de traitement d'une tumeur osseuse comprenant l'administration à une personne en ayant besoin d'une quantité efficace d'un composé de formule (I) tel que décrit ci-dessus ou d'un sel pharmaceutiquement acceptable de celui-ci.

La tumeur osseuse pourra être en particulier une tumeur osseuse primitive comme un ostéosarcome, un chondrosarcome, une tumeur à cellules géantes ou un sarcome d'Ewing ; des métastases osseuses ; ou un myélome multiple.

La présente invention concerne également une composition pharmaceutique comprenant au moins un composé de formule (I), tel que décrit précédemment, ou un sel pharmaceutiquement acceptable de celui-ci et au moins un véhicule pharmaceutiquement acceptable.

Cette composition pourra être formulée de manière à permettre son administration, notamment, par voie parentérale (par ex. sous-cutanée, intraveineuse, intradermique ou intramusculaire), c'est-à-dire de préférence sous forme d'une solution injectable, et destinée aux mammifères, y compris l'homme.

La solution à injecter pourra se trouver sous forme de suspensions aqueuses, de solutions salines isotoniques ou de solutions stériles et injectables qui contiennent des agents de dispersion et/ou des agents mouillants pharmaceutiquement acceptables.

La posologie variera selon le traitement et selon l'affection en cause. Les composés de l'invention en tant que principes actifs peuvent être en particulier utilisés à des doses comprises entre 0,01 mg et 5000 mg par jour, donnés en une seule dose une fois par jour ou administrés en plusieurs doses tout au long de la journée, par exemple deux fois par jour en doses égales. Il peut être nécessaire d'utiliser des doses sortant de ces gammes ce dont l'homme du métier peut se rendre compte lui-même.

La présente invention a également pour objet une composition pharmaceutique telle que définie ci-dessus pour son utilisation dans le traitement d'une tumeur osseuse, notamment telle que définie ci-dessus.

La présente invention concerne également des procédés de préparation d'un composé de formule (I) selon l'invention.

Un premier procédé comprend les étapes suivantes :
(a) réaction de la doxorubicine ou un sel de celle-ci avec un composé de formule (A) suivante : 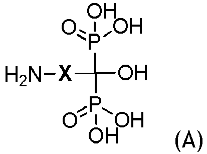
   ou un sel de celui-ci,
   dans laquelle **X** est tel que défini ci-dessus,
   pour donner un composé de formule (I) selon l'invention, et
(b) éventuellement salification du composé de formule (I) obtenu à l'étape (a) précédente pour donner un sel pharmaceutiquement acceptable de celui-ci.

### Etape (a) :

La doxorubicine pourra être utilisée en particulier sous forme de son chlorhydrate.

Le composé de formule (A) peut être préparé comme décrit dans la partie expérimentale ci-dessous.

Le couplage entre la doxorubicine ou un sel de celle-ci et le composé de formule (A) ou un sel de celui-ci pourra être réalisé dans un solvant choisi parmi le tétrahydrofurane (THF), le méthanol, le diméthylsulfoxyde (DMSO), l'eau et leurs mélanges, notamment dans un mélange eau/THF. Ce couplage pourra être réalisé à une température comprise entre 0 et 80°C, en particulier à température ambiante.

Par « température ambiante », on entend, au sens de la présente invention, une température comprise entre 15 et 40°C, de préférence entre 20 et 30°C, notamment d'environ 25°C.

### Etape (b) :

La salification du composé de formule (I) pourra être réalisée par réaction du composé de formule (I) avec un acide ou une base pharmaceutiquement acceptable.

Un second procédé destiné à préparer des composés de formule (I) pour lequel X représente un groupe (1) comprend les étapes suivantes :
(i) réaction de couplage d'un composé de formule (B) suivante : 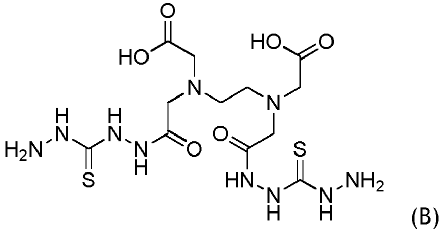 ou un sel de celui-ci,
   avec un composé de formule (C) suivante : 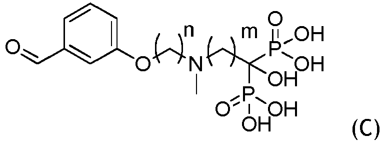
   ou un sel de celui-ci,
   dans laquelle n et m sont tels que définis précédemment,
   et la doxorubicine ou un sel de celle-ci,
   la réaction de couplage étant effectuée en une seule étape en présence du composé de formule (B) ou d'un sel de celui-ci, du composé de formule (C) ou d'un sel de celui-ci et de la doxorubicine ou d'un sel de celle-ci ou en deux étapes par couplage préalable du composé de formule (B) ou d'un sel de celui-ci avec la doxorubicine ou un sel de celle-ci avant d'effectuer le couplage avec le composé de formule (C) ou un sel de celui-ci, ou par couplage préalable du composé de formule (B) ou d'un sel de celui-ci avec le composé de formule (C) ou un sel de celui-ci avant d'effectuer le couplage avec la doxorubicine ou un sel de celle-ci,
   pour donner un composé de formule (I) selon l'invention pour lequel **X** représente un groupe (1), et
(ii) éventuellement salification du composé de formule (I) obtenu à l'étape (i) précédente pour donner un sel pharmaceutiquement acceptable de celui-ci.

### Etape (i) :

La doxorubicine pourra être utilisée en particulier sous forme de son chlorhydrate.

Le composé de formule (B) peut être préparé comme décrit dans la partie expérimentale ci-dessous par réaction du thiocarbohydrazide (composé **2**) avec du dianhydride d'éthylènediaminetétraacétique (composé **1**).

Le composé de formule (C) peut être préparé comme décrit dans la demande WO 2012/130911. Il pourra être utilisé en particulier sous forme d'un sel disodique.

Selon une première variante, les composés de formules (B) et (C) et la doxorubicine ou un sel de ceux-ci sont mis à réagir tous ensemble pour donner un composé de formule (I) selon l'invention pour lequel **X** représente un groupe (1) (réaction de couplage en 1 étape).

Selon une deuxième variante (réaction de couplage en 2 étapes), le composé de formule (B) ou un sel de celui-ci est mis à réagir avec la doxorubicine ou un sel de celle-ci dans une première étape pour donner le composé intermédiaire de formule (D) suivante : 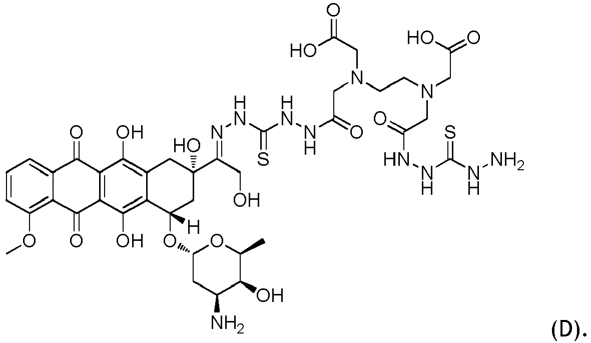 Ce composé de formule (D) est alors mis à réagir avec le composé de formule (C) ou un sel de celui-ci dans une seconde étape pour donner un composé de formule (I) selon l'invention pour lequel X représente un groupe (1).

Selon une troisième variante (réaction de couplage en 2 étapes), le composé de formule (B) ou un sel de celui-ci est mis à réagir avec le composé de formule (C) ou un sel de celui-ci dans une première étape pour donner le composé intermédiaire de formule (E) suivante : 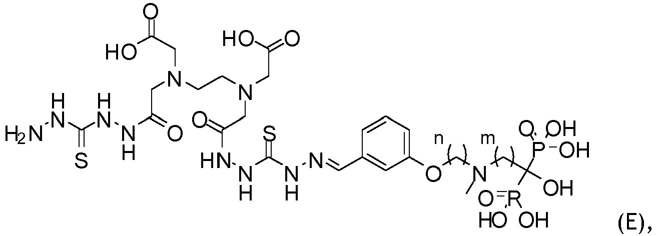 dans laquelle n et m sont tels que définis précédemment.
Ce composé de formule (E) est alors mis à réagir avec la doxorubicine ou un sel de celle-ci dans une seconde étape pour donner un composé de formule (I) selon l'invention pour lequel **X** représente un groupe (1).

Les réactions de couplage mentionnées ci-dessus dans les trois variantes pourront être réalisées en milieu acide, notamment en présence d'acide trifluoroacétique. Un solvant choisi parmi le tétrahydrofurane, le méthanol, le diméthylsulfoxyde, l'eau et leurs mélanges pourra être utilisé, en particulier un mélange THF/eau. Ce couplage pourra être réalisé à température ambiante.

Par « température ambiante », on entend, au sens de la présente invention, une température comprise entre 15 et 40°C, de préférence entre 20 et 30°C, notamment d'environ 25°C.

### Etape (ii) :

La salification du composé de formule (I) pourra être réalisée par réaction du composé de formule (I) avec un acide ou une base pharmaceutiquement acceptable.

Le composé obtenu par l'un des procédés décrits ci-dessus pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation ou précipitation si le composé est cristallin, par chromatographie sur colonne (par ex. sur gel de silice, la silice pouvant éventuellement être modifiée notamment pour la rendre hydrophobe comme sur une colonne C18 ou C8) ou encore par chromatographie liquide haute performance (HPLC).

La présente invention a également pour objet un composé de formule (A) ou (B) telle que définie ci-dessus ou un sel de celui-ci, tel qu'un sel pharmaceutiquement acceptable défini ci-dessus.

Ce composé pourra être choisi en particulier parmi les composés 4, 7, 13 décrits dans la partie expérimentale ci-dessous et les sels de ceux-ci, tels que des sels pharmaceutiquement acceptables définis ci-dessus.

De tels composés sont utiles comme intermédiaires de synthèse, en particulier pour préparer les composés de formule (I) tels que définis ci-dessus.

La présente invention est illustrée par les exemples non limitatifs qui suivent.

### FIGURES

La **Figure 1** représente l'évolution en fonction du temps du volume tumoral moyen (mm³) d'une tumeur HOS développée chez des souris dans les groupes contrôle (CT), composé **(II),** composé **(III),** doxorubicine, vecteur **5** et doxorubicine + vecteur**5.**
La **Figure 2** représente les radiographies à J27 des pattes tumorales des 10 souris de chacun des groupes contrôle (CT), composé **(II)** et composé **(III).**

### EXEMPLES

### Abréviations utilisées :

- DMSO: : Diméthylsulfoxyde
- DPBS: : Dulbecco's Phosphate-Buffered Saline
- EDTA: : Acide éthylène diamine tétraacétique
- ES: : Electrospray
- HPLC: : Chromatographie liquide haute performance
- MS: : Spectromètre de masse
- rdt: : Rendement
- RMN: : Résonance magnétique nucléaire
- sat: : Saturé
- TA: : Température ambiante
- TFA: : Acide trifluoroacétique
- THF: : Tétrahydrofurane
- TRIS :: 2-Amino-2-(hydroxymethyl)propane-1,3-diol

### 1. Synthèse des composés selon l'invention

### Composé (II) :

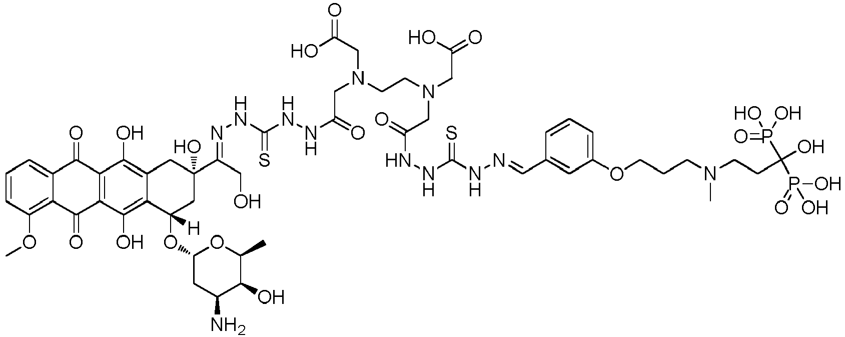

### Etape 1 :

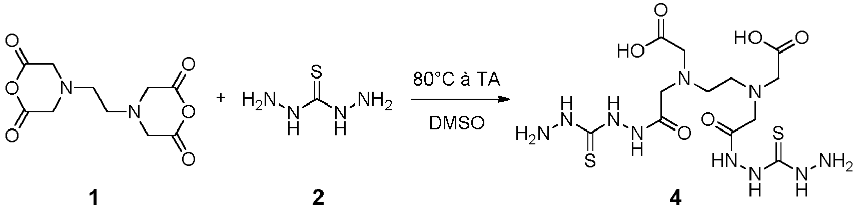

Le composé **1** (3 g, 11.7 mmol) est solubilisé dans du DMSO (15 ml) à 80°C puis est ajouté à une solution de composé **2** (5 g, 47.2 mmol) dans du DMSO (30 ml) à 80°C. La solution transparente obtenue est agitée 3 h à TA. Le mélange réactionnel est dilué avec un mélange de méthanol (120 ml) et d'eau (120 ml). A la solution transparente obtenue, du méthanol (600 ml) est ajouté en agitant suivi d'éther diéthylique (150 ml) et l'agitation est poursuivie pendant 5 min à TA. Le précipité obtenu est filtré et lavé avec du méthanol, puis solubilisé complètement dans 150 ml d'eau à TA pendant 15 min. A la solution obtenue, du méthanol (600 ml) est rajouté, suivi d'éther diéthylique (150 ml) et l'agitation est continuée pendant 5 min à TA. Le précipité obtenu est filtré, lavé avec du méthanol suivi d'éther diéthylique, puis séché sous vide à TA. Le composé **4** (3.2 g, rdt = 58%) est obtenu. Spectre RMN ¹H (D₂O, 400 MHz) δ, ppm: 3.87 (2H, s), 3.70 (2H, s), 3.24 (2H, s).

### Etape 2 :

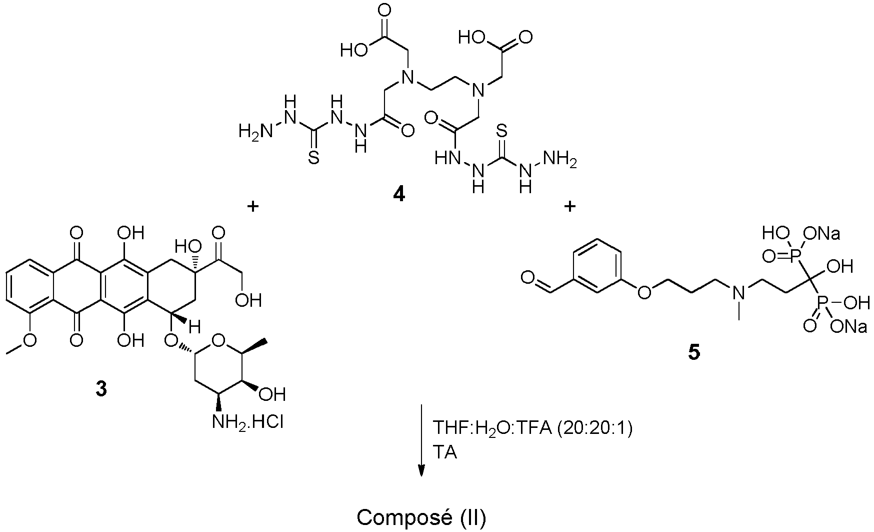

La synthèse du composé **5** est décrite dans la demande WO 2012/130911.
Sous argon, une solution du composé **4** (1060 mg, 2.26 mmol) dans 24 ml d'un mélange THF/H₂O/TFA (20/20/1) est ajoutée en une fois à une solution des composés **3** (918 mg, 1.58 mmol) et **5** (1028 mg, 2.26 mmol) dans 24 ml du mélange THF/H₂O/TFA (20/20/1). La solution rouge foncée obtenue est agitée à TA pendant 16h à l'abri de la lumière. 360 ml d'un mélange Et₂O/MeOH (5/1) sont ensuite ajoutés à la solution en maintenant l'agitation et la suspension de précipité rouge obtenu est agitée pendant 5 min à TA. Le précipité est filtré et lavé avec Et₂O puis séché sous vide. La poudre rouge obtenue est solubilisée dans 10 ml de mélange NaHCO₃sat : H₂O (1:1) et la solution obtenue est introduite dans une colonne C18 puis éluée avec un gradient de [600 ml de 3% MeOH/H₂O + 2 ml de 20% NH₃/H₂O] à [600 ml de 50% MeOH/H₂O + 2 ml de 20% NH₃/H₂O]. Le produit final sort à partir d'environ 450 ml d'éluant dans environ 150 ml d'éluant. Les fractions contenant le produit final sont évaporées sous vide jusqu'à environ 10 ml de solution restante qui est ensuite diluée avec MeOH (40 ml) suivi de Et₂O (200 ml). Le mélange obtenu est agité jusqu'à la formation d'un précipité rouge qui est agité pendant 5 min à TA, puis filtré, lavé avec Et₂O, et séché sous vide à TA. Le composé (II) (526 mg, rdt = 23%) est obtenu.

Spectre RMN ¹H (1%TFA-D/DMSO-D6, 400 MHz) δ, ppm: 14.07 (1H, s), 13.36 (1H, s), 11.97 (1H, s), 11.24 (1H, s), 10.53 (1H, s), 10.48 (1H, s), 10.30 (1H, s), 9.80 (1H, s), 8.05 (1H, s), 7.95 (2H, s), 7.84 (2H, m), 7.68 (1H, dd), 7.46 (1H, s), 7.40 (1H, d), 7.33 (1H, t), 7.02 (1H, d), 5.30 (1H, s), 4.99 (1H, s), 4.61 (1H, t), 4.5-3.7 (12H, m), 3.6-2.85 (14H, m), 3.01 (1H, d), 2.81 (3H, s), 2.27 (4H, m), 2.13 (2H, m), 1.85 (1H, t), 1.65 (1H, d), 1.18 (3H, d). Spectre RMN ³¹P (1%TFA-D/DMSO-D6, 162 MHz) δ, ppm: 18.23. Spectre de masse (ES-) (m/z): [M-H]⁻ 1386. Pureté par analyse HPLC (C18, H₂O/EDTA/NH₃-MeCN): 97%.

### Composé (III) :

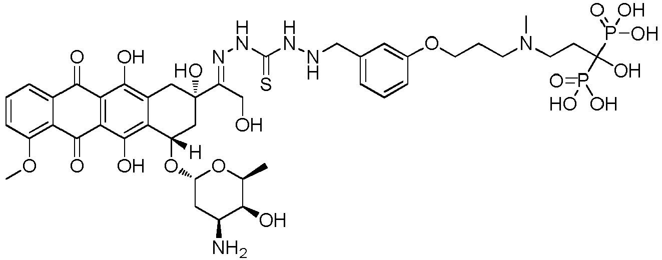

### Etape 1 :

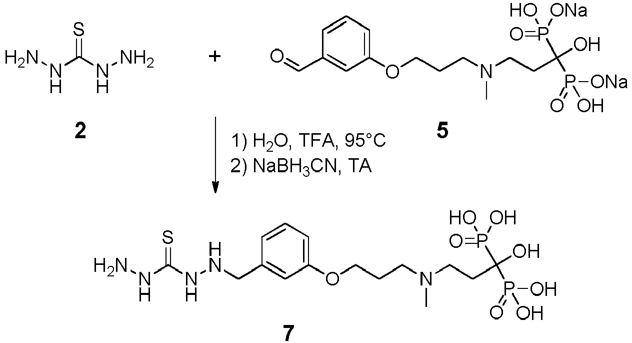

Sous agitation, le composé **5** (1 g, 2.2 mmol) est ajouté à une solution de composé **2** (1 g, 9.4 mmol) dans l'eau (40 ml) à 95°C. A la solution transparente obtenue, du TFA (0.8 ml) est additionné et le mélange est agité 30 min à la même température. Le mélange est mis ensuite à TA et du NaBH₃CN (0.6 g, 9.5 mmol) est ajouté, puis l'agitation est poursuivie pendant 24 h à TA. Le mélange réactionnel est introduit sur une colonne C18 qui est éluée avec un gradient de [600 ml de 3% MeOH/H₂O] à [600 ml de 50% MeOH/H₂O]. L'excès de composé **2** est élué après environ 150 ml d'éluant et le produit final **7** est élué après environ 400 ml d'éluant dans un volume d'environ 200 ml d'éluant. Les fractions sont évaporées sous vide jusqu'à environ 10 ml de solution restante qui est ensuite diluée en agitant avec du MeOH (200 ml) suivi de Et₂O (300 ml). Le précipité obtenu est filtré, lavé avec Et₂O et séché sous vide à TA. Le composé **7** (780 mg, rdt = 71%) est obtenu.

Spectre RMN ¹H (D2O, 400 MHz) δ, ppm: 7.26 (1H, t), 6.97 (2H, m), 6.89 (1H, dd), 4.10 (2H, t), 3.89 (2H, s), 3.60-3.10 (4H, s), 2.82 (3H, s), 2.31 (2H, m), 2.19 (2H, m). Spectre RMN ³¹P (D₂O, 162 MHz) δ, ppm: 16.87. Spectre de masse (ES-) (m/z): [M-H]⁻ 500.

### Etape 2 :

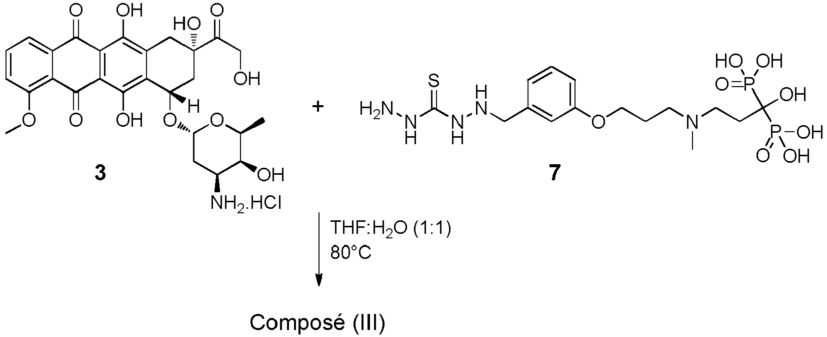

Le mélange des composés **7** (250 mg, 0.5 mmol) et **3** (290 mg, 0.5 mmol) est solubilisé dans **6** ml d'un mélange THF/H₂O (1/1) à 80°C, agité à cette température pendant 3 min, puis évaporé à sec sous vide. Le mélange sec obtenu est laissé pendant 5 min à 80°C sous vide et le ballon bouché est refroidi rapidement à TA avec de l'eau froide. Le solide obtenu est solubilisé sous agitation à 30-40°C dans un mélange de NaHCO₃sat (4 ml), d'eau (4 ml) et de THF (8 ml). La solution transparente très foncée obtenue est diluée avec de l'eau (50 ml) et introduite sur une colonne C18. La colonne est éluée avec un gradient de 600 ml de 3% MeOH/H₂O à 600 ml MeOH. Le composé **(III)** est élué après environ 400 ml d'éluant dans un volume d'environ 200 ml d'éluant. Les fractions sont évaporées sous vide jusqu'à environ 10 ml de solution restante qui est diluée en agitant avec du MeOH (100 ml) suivi de Et₂O (300 ml) Le précipité rouge formé est filtré, lavé avec Et₂O et séché sous vide à TA. Le composé **(III)** (160 mg, rdt = 32%) est obtenu.

Spectre RMN ¹H (D₂O/THF-D8=3/1, 400 MHz) δ, ppm: 8.03 (2H, m), 7.81 (1H, d), 7.40 (1H, t), 7.21 (1H, s), 7.07 (1H, d), 5.40 (1H, s), 5.17 (1H, t), 4.42-3.99 (11H, m), 3.80-3.35 (6H, m), 3.14-3.95 (4H, m), 2.73-2.35 (7H, m), 2.22 (1H, t), 2.02 (1H, m), 1.47 (1H, m), 1.44 (3H, d). Spectre RMN ³¹P (D₂O/THF-D8=3/1, 162 MHz) δ, ppm: 14.62. Spectre de masse (ES-) (m/z): [M-H]⁻ 1025. Pureté par analyse HPLC (C18, H₂O/EDTA/NH₃-MeCN) : 98%.

### Composé (IV) :

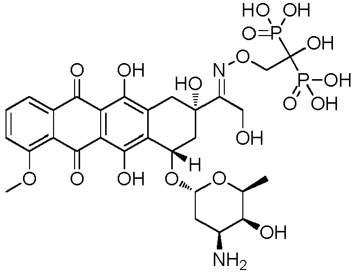

### Etape 1 :

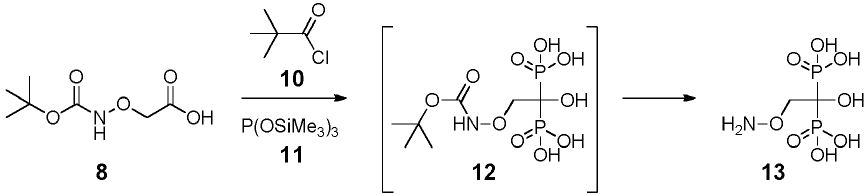

A une solution transparente de produit commercial **8** (300 mg, 1.57 mmol) dans du THF (2 ml), de la triéthylamine (218 µl, 1.57 mmol) est ajoutée et le mélange homogène est refroidi à 0°C. A cette température, la solution de produit **10** (190 mg, 1.57 mmol) dans du THF (600 µl) est ajoutée progressivement et le mélange réactionnel est agité 15 min à 0°C. Le précipité est filtré rapidement afin de collecter la solution transparente sous argon. A cette solution, le composé **11** (1.4 g, 4.71 mmol) est ajouté et le mélange transparent obtenu est agité pendant 1 h à TA. Du méthanol (3 ml) est rajouté et après 10 min d'agitation le mélange est concentré sous vide à sec à 36°C. Au résidu obtenu, du TFA (2 ml) est ajouté et le précipité blanc formé est agité à TA pendant 1 h, puis les volatiles sont évaporés à sec à 36°C. L'huile obtenue est traitée avec du méthanol suivi d'éther et le précipité blanc formé est filtré, lavé avec de l'éther et séché. Le composé **13** est obtenu (205 mg, rdt = 55%).

Spectre RMN ¹H (D₂O, 400 MHz) δ, ppm: 4.49 (2H, t). Spectre RMN ³¹P (D₂O, 162 MHz) δ, ppm: 14.18.

### Etape 2 :

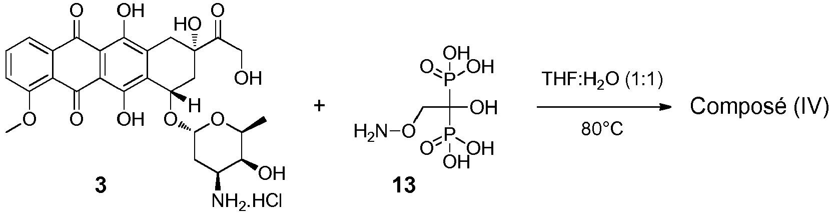

Sous argon, une solution des composés **3** (270 mg, 0.46 mmol) et **13** (100 mg, 0.42 mmol) dans 3 ml d'un mélange THF/H₂O/TFA (20/20/1) est agitée à TA pendant 16h à l'abri de la lumière. 20 ml d'un mélange NaHCO₃sat : H₂O (1:1) est rajouté et la solution obtenue est introduite dans une colonne C18 puis éluée avec un gradient de [600 ml de 3% MeOH/H₂O + 2 ml de 20% NH₃/H₂O] à [600 ml de 50% MeOH/H₂O + 2 ml de 20% NH₃/H₂O]. Le produit final sort à partir d'environ 210 ml d'éluant dans environ 150 ml d'éluant. Les fractions contenant le produit final sont évaporées sous vide jusqu'à environ 5 ml de solution restante qui est ensuite diluée avec du méthanol (40 ml) puis de l'éther diéthylique (200 ml). Le mélange obtenu est agité jusqu'à la formation d'un précipité rouge qui est agité pendant 5 min à TA, puis filtré, lavé avec de l'éther diéthylique, et séché sous vide à TA. Le composé **(IV)** (160 mg, rdt = 47%) est obtenu.

Spectre RMN ¹H (D₂O, 400 MHz) δ, ppm: 7.66 (1H, t), 7.38 (2H, m), 5.48 (1H, s), 4.84 (1H, s), 4.62 (2H, m), 4.40 (2H, s), 4.29 (1H, m), 3.90 (3H, s), 3.87 (1H, s), 3.73 (1H, t), 2.90 (1H, d), 2.57 (1H, d), 2.35 (1H, d), 2.15-1.92 (3H, m), 1.33 (3H, d). Spectre RMN ³¹P (D₂O, 162 MHz) δ, ppm: 15.19 (1P, d), 15.08 (1P, d). Spectre de masse (ES+) (m/z): [M-H]⁻ 807. Pureté par analyse HPLC (C18, H₂O/EDTA/NH₃-MeCN): 99%.

### 2. Solubilité des composés (II), (III) et (IV) comparée aux produits (a) et (b).

La demande WO 2012/130911 décrit le dérivé (b) ci-dessous résultant du couplage entre un motif doxorubicine modifiée et un vecteur de type HBP. Ce composé (b) est insoluble dans l'eau et peu soluble dans l'eau additionnée de TRIS ce qui rend difficile l'utilisation d'un tel composé.

En utilisant le même vecteur HBP que dans la demande WO 2012/130911, le produit (a) issu du couplage de la doxorubicine avec ce vecteur **5** est également insoluble dans l'eau, dans l'eau avec des additifs organiques (Tween®, PEG, glycérine, glucose, TRIS) ou minéraux (carbonate ou bicarbonate de sodium), et dans les solvants organiques (l'éthanol ou diméthylsuloxyde ou leur mélanges avec de l'eau), ce qui rend difficile l'exploitation de ce produit.

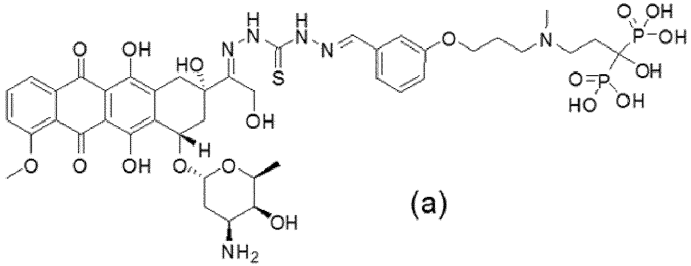

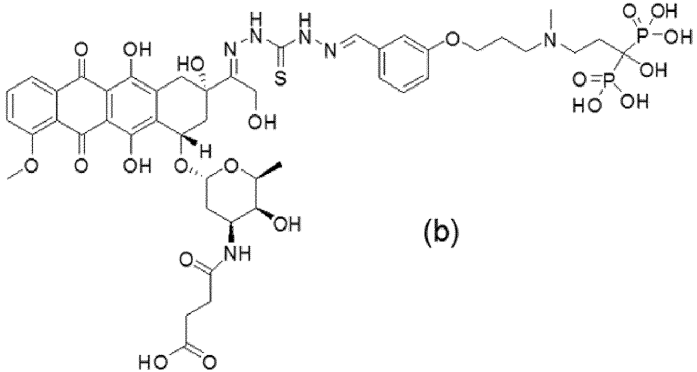

La solubilité des produits (II), (III) et (IV) selon l'invention est fortement améliorée par rapport à la solubilité des molécules (a) et (b) (voir tableau ci-dessous), ce qui permet de faciliter l'utilisation de tels composés comme médicament.

| | (II) | (III) | (IV) | (a) | (b) |
|---|---|---|---|---|---|
| DPBS ou eau | autour de 255 mM | autour de 160 mM | autour de 315 mM | insoluble | insoluble |
| solution de TRIS dans l'eau à 2 mg/ml | non testée | | | insoluble | autour de 1 mM |

### 3. Tests biologiques

### Matériel et Méthodes :

L'activité anti-tumorale des composés selon l'invention **(II)** et **(III)** a été évaluée dans un modèle murin xénogénique orthotopique d'ostéosarcome ostéolytique induit par injection de la lignée tumorale humaine HOS (ostéosarcome humain, ATCC) en paratibial chez des souris immunodéprimées. L'activité des molécules selon l'invention a été comparée aux deux sous-unités principales les composant (la doxorubicine et le vecteur **5)** seules ou en association. Les traitements, sur 10 animaux par groupe, ont débuté le lendemain de l'induction tumorale et ont été administrés 1 à 2 fois par semaine pendant 5 semaines par voie intrapéritonéale. Les volumes tumoraux ont été mesurés deux fois par semaine à l'aide d'un pied à coulisse et un suivi radiographique a été réalisé une fois par semaine. Le volume tumoral, exprimé en mm³ est calculé selon la formule suivante : V=(Lxl²)/2 (L et l correspondent respectivement à la grande longueur et à la petite longueur de la tumeur exprimées en mm).

Les groupes de traitement suivants ont donc été étudiés, chacun sur 10 souris :

| **Groupes** | | **Traitement administré par voie intrapéritonéale.** (Volume d'injection de 10mL/kg) |
|---|---|---|
| 1 | Contrôle (solvant : DPBS) | 2x/sem |
| 2 | Composé (II) (2,5 mM dans DPBS) | 25 µmol/kg - 2x/sem |
| 3 | Composé (III) (2,5 mM dans DPBS) | 25 µmol/kg - 2x/sem |
| 4 | Doxorubicine (0,37 mM dans NaCl_{aq} 0,9%) | 3,7 µmol/kg - 1x/sem |
| 5 | Vecteur **5** (2,5 mM dans DPBS) | 25 µmol/kg - 2x/sem |
| 6 | Doxorubicine (0,37 mM dans NaCl_{aq} 0,9%) | 3,7 µmol/kg - 1x/sem |
| | Vecteur **5** (2,5 mM dans DPBS) | 25 µmol/kg - 2x/sem |

### Résultats :

*Toxicité.* Administrée à une dose de 12mg/kg (20 µmol/kg), la doxorubicine est responsable d'un amaigrissement très précoce des animaux (perte de poids>10% nécessitant l'euthanasie des animaux après 2 semaines de traitement) et d'une cardiotoxicité. Les composés **(II), (III)** et **(IV)** utilisés à une dose molaire équivalente n'induisent pas ces signes de toxicité. Dans le but de traiter plus efficacement la tumeur, les molécules bifonctionnelles peuvent même être utilisées à des doses plus importantes que les anticancéreux seuls (sachant que la dose usuelle de traitement par la doxorubicine est de 2mg/kg (3,7µmol/kg)). Une étude de détermination de la dose maximale utilisable a ainsi mis en évidence une perte de poids similaire des souris lorsque les composés **(II), (III)** et **(IV)** étaient utilisés à une dose 13 fois plus importante que la doxorubicine seule par voie intra-veineuse ou par voie intrapéritonéale.

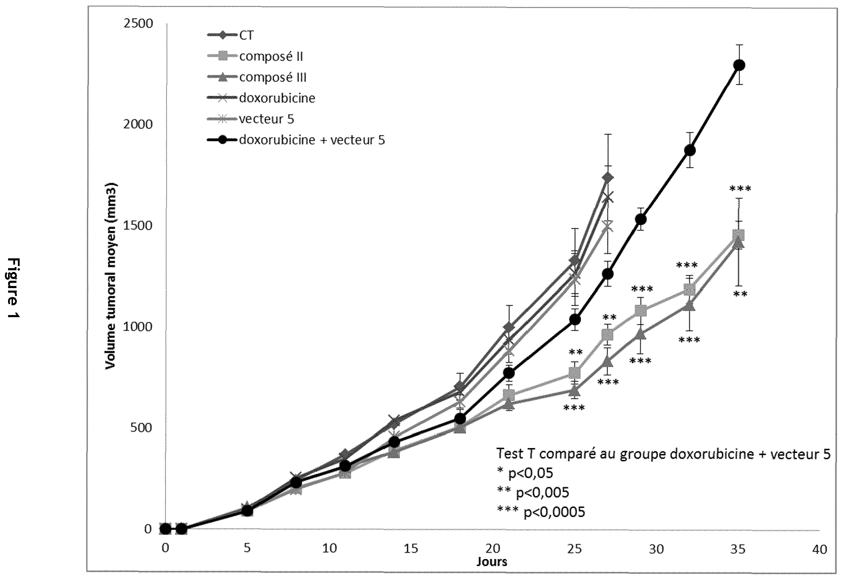

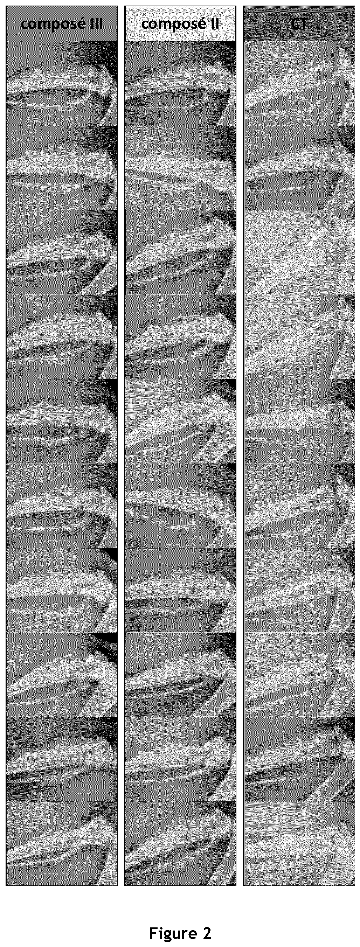

*Propriétés anti-tumorales. (voir* *Figures 1* *et* *2**)* Les composés **(II)** et **(III)** réduisent significativement la taille des tumeurs extra-osseuses mesurées par rapport au groupe contrôle et aux groupes traités avec le vecteur **5** et/ou la doxorubicine.

La comparaison des volumes tumoraux des différents groupes est possible cependant jusqu'à J27. Après cette date, les animaux des groupes contrôle, doxorubicine et vecteur **5** ont dû être euthanasiés en raison de volumes tumoraux trop importants. En outre, à J32, tous les animaux du groupe doxorubicine + vecteur **5** présentent une tumeur d'un volume supérieur à 1500 mm³ tandis que seule une souris du groupe composé **(II)** dépasse ce seuil.

*Prévention des lésions osseuses associées au développement tumoral.* Les composés **(II)** et **(III)** réduisent les fractures métaphysaires, l'altération de l'intégrité des corticales et la néoformation d'os ectopique par rapport aux groupes contrôle, doxorubicine, vecteur **5** et doxorubicine + vecteur **5.** A J27, alors que 90% des animaux du groupe contrôle présentent des fractures au niveau de la métaphyse tibiale, siège du développement tumoral, ces fractures ne sont détectées chez aucune des souris traitées par les composés (II) et (III). Les lésions observées dans le groupe doxorubicine sont équivalentes à celles du groupe contrôle (ostéolyses importantes à la surface des corticales et fractures métaphysaires). Le vecteur **5** (groupes vecteur **5** et doxorubicine + vecteur **5)** réduit en partie les fractures métaphysaires mais des lésions ostéolytiques importantes sur les surfaces des corticales sont à déplorer. En revanche, les composés selon l'invention préviennent significativement l'ostéolyse associée au développement tumoral dans ce modèle d'ostéosarcome.

## Revendications

1. Composé de formule générale (I) suivante : 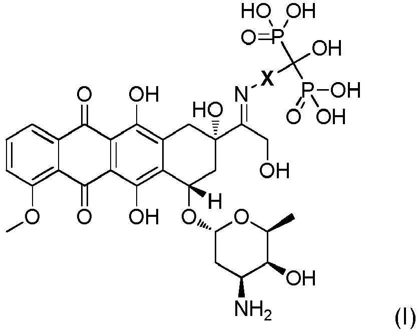 ou un sel pharmaceutiquement acceptable de celui-ci,
dans laquelle **X** représente un groupe bivalent choisi parmi :
(1) 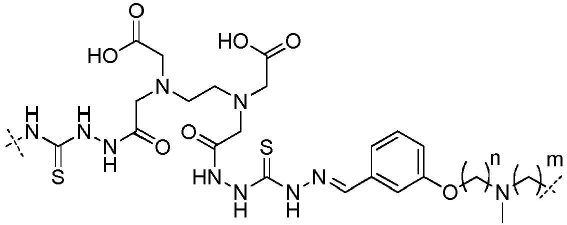 avec n et m représentant chacun, indépendamment l'un de l'autre, un nombre entier compris entre 1 et **6,**
(2) 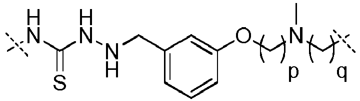 avec p et q représentant chacun, indépendamment l'un de l'autre, un nombre entier compris entre 1 et 6, et
(3) 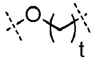 avec t représentant un nombre entier compris entre 1 et 6,
ces groupes étant liés à la fonction imine du composé de formule (I) via leur atome d'azote ou d'oxygène terminal et à la fonction acide hydroxybisphosphonique du composé de formule (I) via leur atome de carbone terminal.

2. Composé selon la revendication 1, **caractérisé en ce que** n, m, p, q et t représentent chacun, indépendamment les uns des autres, un nombre entier compris entre 1 et 4.

3. Composé selon la revendication 1, **caractérisé en ce qu'**il est choisi parmi les composés suivants : 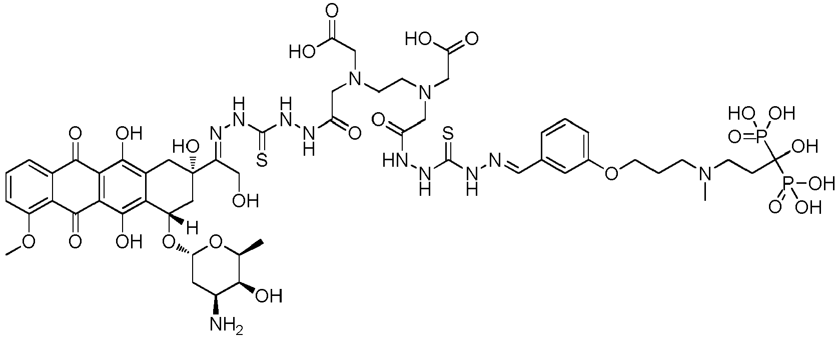 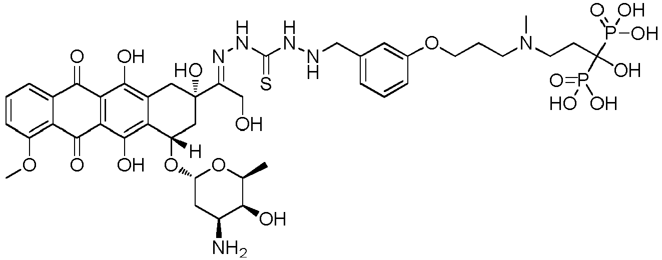 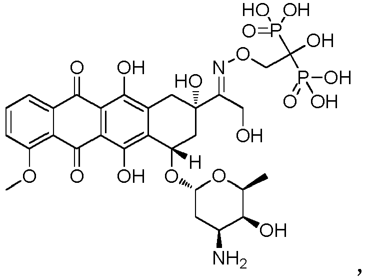 et leurs sels pharmaceutiquement acceptables.

4. Composé selon l'une quelconque des revendications 1 à 3 pour son utilisation en tant que médicament.

5. Composé selon l'une quelconque des revendications 1 à 3 pour son utilisation dans le traitement d'une tumeur osseuse.

6. Composé pour son utilisation selon la revendication 5, **caractérisé en ce que** la tumeur osseuse est choisi parmi les tumeurs osseuses primitives comme un ostéosarcome, un chondrosarcome, une tumeur à cellules géantes ou un sarcome d'Ewing ; les métastases osseuses ; et le myélome multiple.

7. Composition pharmaceutique comprenant au moins un composé de formule (I) selon l'une quelconque des revendications 1 à 3 et au moins un véhicule pharmaceutiquement acceptable.

8. Composition selon la revendication 7 pour son utilisation en tant que médicament.

9. Composition selon la revendication 7 pour son utilisation dans le traitement d'une tumeur osseuse.

10. Composition pour son utilisation selon la revendication 9, **caractérisée en ce que** la tumeur osseuse est choisie parmi les tumeurs osseuses primitives comme un ostéosarcome, un chondrosarcome, une tumeur à cellules géantes ou un sarcome d'Ewing ; les métastases osseuses ; et le myélome multiple.

11. Procédé de préparation d'un composé selon l'une quelconque des revendications 1 à 3 comprenant les étapes suivantes :
(a) réaction de la doxorubicine ou un sel de celle-ci avec un composé de formule (A) suivante : 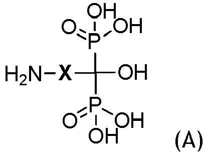 ou un sel de celui-ci,
dans laquelle X est tel que défini à la revendication 1,
pour donner un composé de formule (I), et
(b) éventuellement salification du composé de formule (I) obtenu à l'étape (a) précédente pour donner un sel pharmaceutiquement acceptable de celui-ci.

12. Procédé de préparation d'un composé selon l'une quelconque des revendications 1 à 3 pour lequel **X** représente un groupe (1) comprenant les étapes suivantes :
(i) réaction de couplage d'un composé de formule (B) suivante : 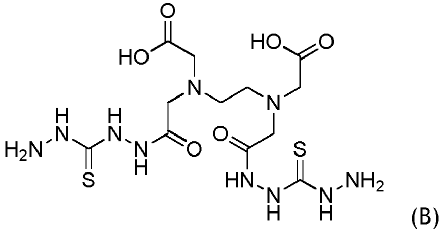 ou un sel de celui-ci,
avec un composé de formule (C) suivante : 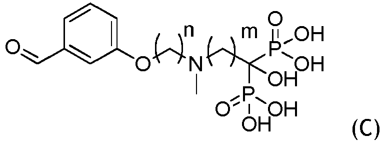 ou un sel de celui-ci,
dans laquelle n et m sont tels que définis à la revendication 1,
et la doxorubicine ou un sel de celle-ci,
la réaction de couplage étant effectuée en une seule étape en présence du composé de formule (B) ou d'un sel de celui-ci, du composé de formule (C) ou d'un sel de celui-ci et de la doxorubicine ou d'un sel de celle-ci ou en deux étapes par couplage préalable du composé de formule (B) ou d'un sel de celui-ci avec la doxorubicine ou un sel de celle-ci avant d'effectuer le couplage avec le composé de formule (C) ou un sel de celui-ci, ou par couplage préalable du composé de formule (B) ou d'un sel de celui-ci avec le composé de formule (C) ou un sel de celui-ci avant d'effectuer le couplage avec la doxorubicine ou un sel de celle-ci,
pour donner un composé de formule (I) pour lequel **X** représente un groupe (1), et
(ii) éventuellement salification du composé de formule (I) obtenu à l'étape (i) précédente pour donner un sel pharmaceutiquement acceptable de celui-ci.

13. Composé de formula (A) ou (B) suivante : 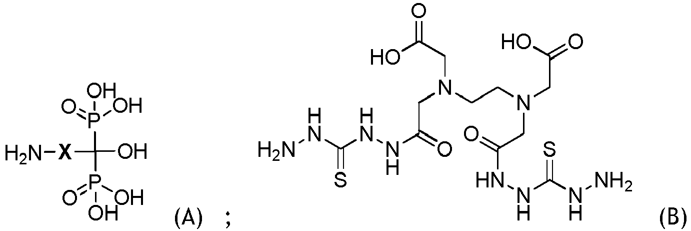 dans laquelle **X** est tel que défini à la revendication 1,
ou un sel de celui-ci.

14. Composé selon la revendication 13, **caractérisé en ce qu'**il est choisi parmi les composés suivants : 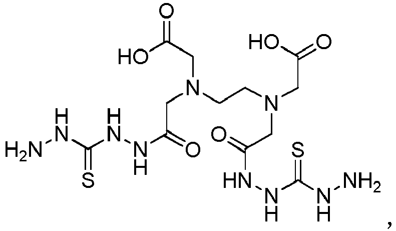 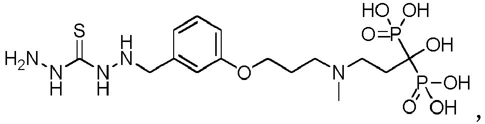 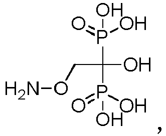 et les sels de ceux-ci.

## Patentansprüche

1. Verbindung der folgenden allgemeinen Formel (I): 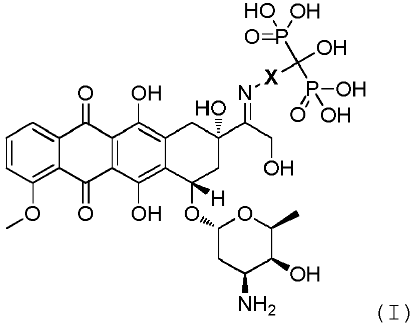 oder ein pharmazeutisch verträgliches Salz davon,
wobei X eine zweiwertige Gruppe darstellt, die ausgewählt ist unter:
(1) 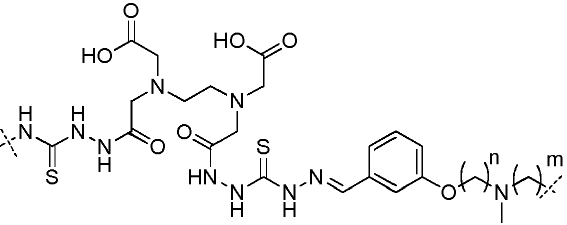 mit n und m, die jeweils unabhängig voneinander eine ganze Zahl zwischen 1 und 6 darstellen,
(2) 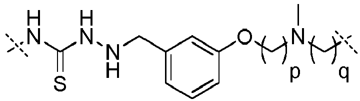 mit p und q, die jeweils unabhängig voneinander eine ganze Zahl zwischen 1 und 6 darstellen, und
(3) 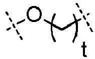 wobei t eine ganze Zahl zwischen 1 und 6 darstellt,
wobei diese Gruppen an die Iminfunktion der Verbindung der Formel (I) über ihr endständiges Stickstoff- oder Sauerstoffatom und an die hydroxybisphosphonische Säurefunktion der Verbindung der Formel (I) über ihr endständiges Kohlenstoffatom gebunden sind.

2. Verbindung nach Anspruch 1, **dadurch gekennzeichnet, dass** n, m, p, q und t jeweils unabhängig voneinander eine ganze Zahl zwischen 1 und 4 darstellen.

3. Verbindung nach Anspruch 1, **dadurch gekennzeichnet, dass** sie unter den folgenden Verbindungen ausgewählt ist: 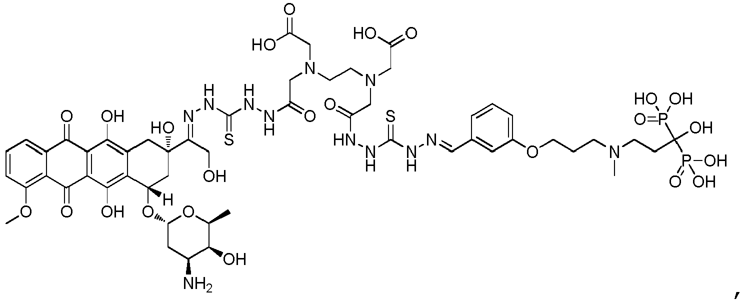 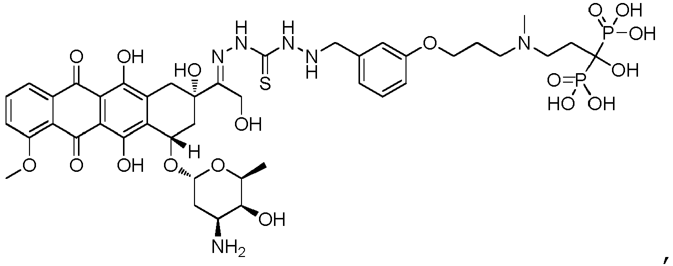 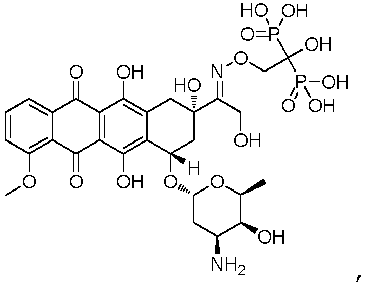 und ihren pharmazeutisch verträglichen Salzen davon.

4. Verbindung nach einem beliebigen der vorstehenden Ansprüche 1 bis 3 zur Verwendung als Arzneimittel.

5. Verbindung nach einem beliebigen der vorstehenden Ansprüche 1 bis 3 zur Verwendung bei der Behandlung eines Knochentumors.

6. Verbindung zur Verwendung nach Anspruch 5, **dadurch gekennzeichnet, dass** der Knochentumor unter primären Knochentumoren wie Osteosarkom, einem Chondrosarkom, einem Riesenzelltumor oder einem Ewing-Sarkom; Knochenmetastasen und multiplem Myelom ausgewählt ist.

7. Pharmazeutische Zusammensetzung, die zumindest eine Verbindung mit der Formel (I) nach einem beliebigen der vorstehenden Ansprüche 1 bis 3 und zumindest einen pharmazeutisch verträglichen Wirkstoff umfasst.

8. Zusammensetzung nach Anspruch 7 zur Verwendung als Arzneimittel.

9. Zusammensetzung nach Anspruch 7 zur Verwendung bei der Behandlung eines Knochentumors.

10. Zusammensetzung zur Verwendung nach Anspruch 9, **dadurch gekennzeichnet, dass** der Knochentumor unter primären Knochentumoren wie einem Osteosarkom, einem Chondrosarkom, einem Riesenzelltumor oder einem Ewing-Sarkom; Knochenmetastasen und multiplem Myelom ausgewählt ist.

11. Verfahren zur Herstellung einer Verbindung nach einem beliebigen der vorstehenden Ansprüche 1 bis 3, das die folgenden Schritte umfasst:
(a) Reaktion des Doxorubicins oder eines Salzes davon mit einer Verbindung der folgenden Formel (A): 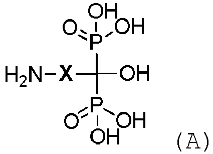 oder ein Salz davon,
wobei X wie in Anspruch 1 definiert ist,
um eine Verbindung der Formel (I) zu ergeben, und
(b) eventuell Salzbildung der Verbindung der Formel (I) bei dem vorherigen Schritt (a), um zu einem pharmazeutisch verträglichen Salz davon zu gelangen.

12. Verfahren zur Herstellung einer Verbindung nach einem beliebigen der vorstehenden Ansprüche 1 bis 3, bei dem X eine Gruppe (1) darstellt, die die folgenden Schritte umfasst:
(i) Kupplungsreaktion einer Verbindung der folgenden Formel (B): 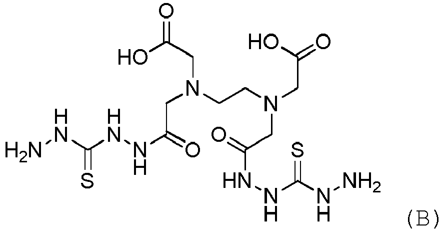 oder ein Salz davon,
mit einer Verbindung der folgenden Formel (C): 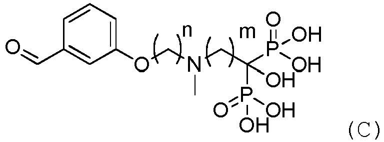 oder ein Salz davon,
wobei n und m wie in Anspruch 1 definiert sind,
und Doxorubicin oder ein Salz davon,
wobei die Kupplungsreaktion in einem einzigen Schritt in Gegenwart der Verbindung der Formel (B) oder eines Salzes davon, der Verbindung der Formel (C) oder eines Salzes davon und Doxorubicin oder eines Salzes davon durchgeführt wird, oder in zwei Schritten durch vorherige Kupplung der Verbindung der Formel (B) oder eines Salzes davon mit Doxorubicin oder einem Salz davon, bevor die Kupplung mit der Verbindung der Formel (C) oder einem Salz davon erfolgt, oder durch vorherige Kupplung der Verbindung der Formel (B) oder einem Salz davon mit der Verbindung der Formel (C) oder einem Salz davon erfolgt, bevor die Kupplung mit dem Doxorubicin oder einem Salz davon durchgeführt wird,
um eine Verbindung der Formel (I) zu ergeben, wobei X eine Gruppe (1) darstellt, und
(ii) eventuell Salzbildung der Verbindung der Formel (I) bei dem vorherigen Schritt (i), um zu einem pharmazeutisch verträglichen Salz davon zu gelangen.

13. Verbindung der folgenden Formel (A) oder (B): 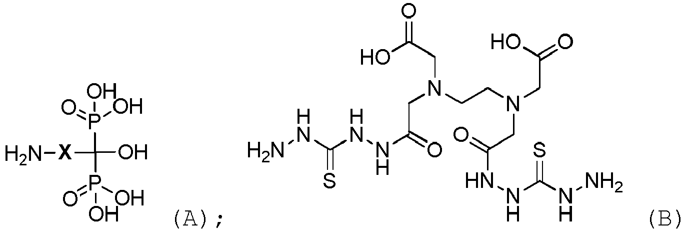 wobei X wie in Anspruch 1 definiert ist,
oder ein Salz davon,

14. Verbindung nach Anspruch 13, **dadurch gekennzeichnet, dass** sie unter den folgenden Verbindungen ausgewählt ist: 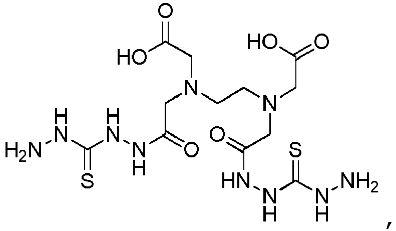 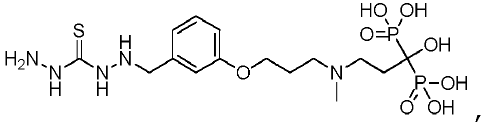 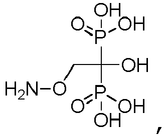 und den Salzen davon.

## Claims

1. A compound of the following general formula (I): 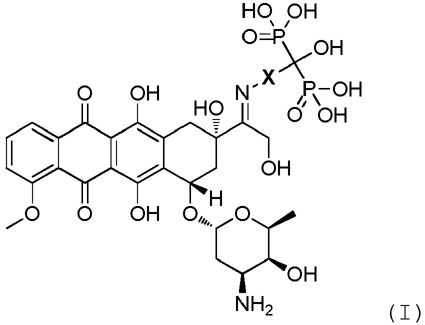 or a pharmaceutically acceptable salt thereof,
wherein X represents a bivalent group selected from:
(1) 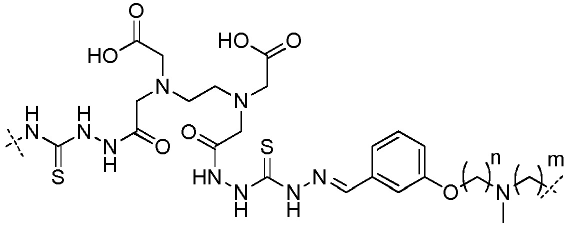 with n and m each independently representing an integer between 1 and 6,
(2) 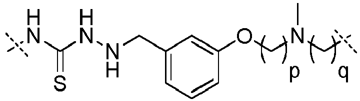 with p and q each independently representing an integer between 1 and 6, and
(3) 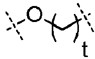 with t representing an integer between 1 and 6,
these groups being bound to the imine function of the compound of formula (I) via their terminal nitrogen or oxygen atom and to the hydroxybisphosphonic acid function of the compound of formula (I) via their terminal carbon atom.

2. The compound according to claim 1, **characterized in that** n, m, p, q and t each independently represent an integer between 1 and 4.

3. The compound according to claim 1, **characterized in that** said compound is selected from the following compounds: 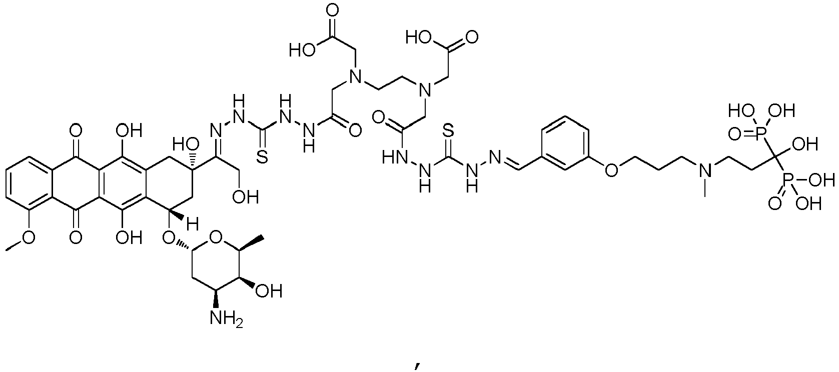 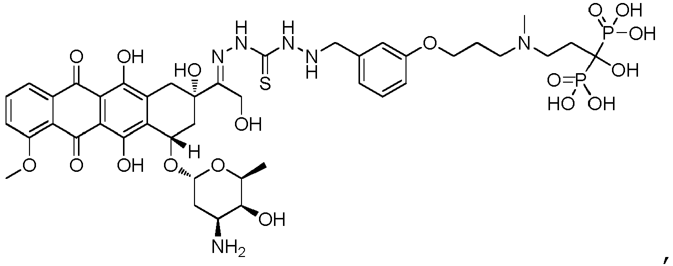 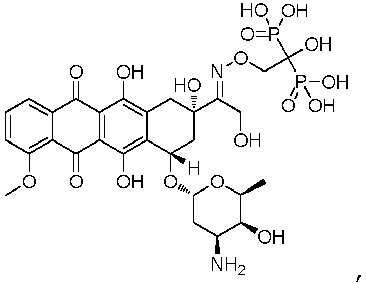 and pharmaceutically acceptable salts thereof.

4. The compound according to any one of claims 1 to 3 for use as a medicinal product.

5. The compound according to any one of claims 1 to 3 for use in the treatment of a bone tumor.

6. The compound for use according to claim 5, **characterized in that** the bone tumor is selected from primary bone tumors such as osteosarcoma, chondrosarcoma, giant cell tumor or Ewing's sarcoma; bone metastases; and multiple myeloma.

7. A pharmaceutical composition comprising at least one compound of formula (I) according to any one of claims 1 to 3 and at least one pharmaceutically acceptable carrier.

8. The composition according to claim 7 for use as a medicinal product.

9. The composition according to claim 7 for use in the treatment of a bone tumor.

10. The composition for use according to claim 9, **characterized in that** the bone tumor is selected from primary bone tumors such as osteosarcoma, chondrosarcoma, giant cell tumor or Ewing's sarcoma; bone metastases; and multiple myeloma.

11. A method for preparing a compound according to any one of claims 1 to 3 comprising the following steps:
(a) reaction of doxorubicin or a salt thereof with a compound of the following formula (A): 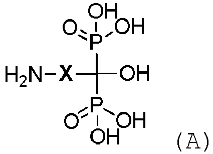 or a salt thereof,
wherein X is as defined in claim 1,
to give a compound of formula (I), and
(b) optionally, salification of the compound of formula (I) obtained in the preceding step (a) to give a pharmaceutically acceptable salt thereof.

12. A method for preparing a compound according to any one of claims 1 to 3 for which X represents a group (1) comprising the following steps:
(i) coupling reaction of a compound of the following formula (B): 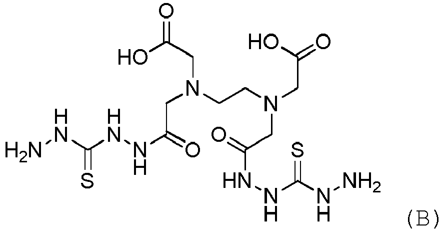 or a salt thereof,
with a compound of the following formula (C): 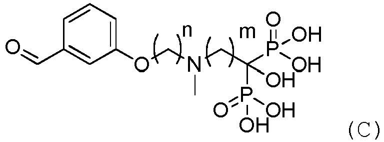 or a salt thereof,
wherein n and m are as defined in claim 1,
and doxorubicin or a salt thereof,
the coupling reaction being carried out in one step in the presence of the compound of formula (B) or a salt thereof, the compound of formula (C) or a salt thereof, and doxorubicin or a salt thereof or in two steps by first coupling the compound of formula (B) or a salt thereof with doxorubicin or a salt thereof before carrying out the coupling with the compound of formula (C) or a salt thereof, or by first coupling the compound of formula (B) or a salt thereof with the compound of formula (C) or a salt thereof before carrying out the coupling with doxorubicin or a salt thereof,
to give a compound of formula (I) for which X represents a group (1), and
(ii) optionally, salification of the compound of formula (I) obtained in the preceding step (i) to give a pharmaceutically acceptable salt thereof.

13. A compound of the following formula (A) or (B): 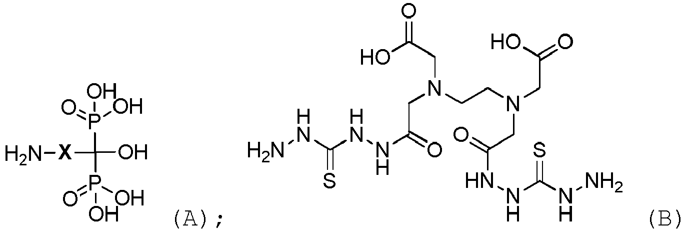 wherein X is as defined in claim 1,
or a salt thereof.

14. The compound according to claim 13, **characterized in that** said compound is selected from the following compounds: 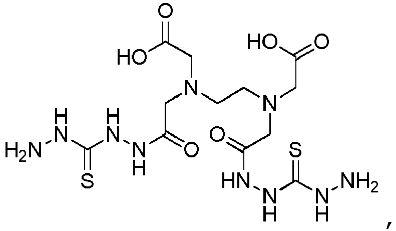 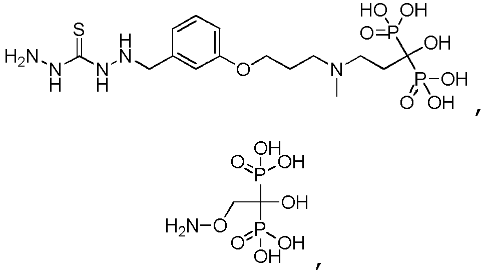 and salts thereof.
